# EUROPÄISCHE PATENTSCHRIFT

(11) **EP 1 957 033 B1**
(45) Veröffentlichungstag und Bekanntmachung des Hinweises auf die Patenterteilung: **08.12.2010**
(21) Anmeldenummer: 07726429.9
(22) Anmeldetag: 20.02.2007
(51) Int. Cl.: A61K 8/58, A61K 8/891, A61K 8/893, A61K 8/894, A61Q 17/04

(54) **KOSMETISCHES SONNENSCHUTZPRODUKT AUF BASIS VON W/SI-EMULSIONEN**
COSMETIC SUN PROTECTION PRODUCT BASED ON W/SI EMULSIONS
PRODUIT COSMÉTIQUE DE PROTECTION SOLAIRE À BASE D'ÉMULSIONS W/SI

(30) Priorität: 21.02.2006 DE 102006008920
(43) Veröffentlichungstag der Anmeldung: 20.08.2008
(73) Patentinhaber: Coty Prestige Lancaster Group GmbH, 55116 Mainz (DE)
(72) Erfinder: GOLZ-BERNER, Karin, MC-98000 Monaco (MC); ZASTROW, Leonhard, MC-98000 Monaco (MC); MITON, Melle, Sandra, F-06300 Nice (FR)
(74) Vertreter: Ziebig, Marlene
(86) Internationale Anmeldenummer: PCT/EP2007/051608
(87) Internationale Veröffentlichungsnummer: WO 2007/096353

(56) Entgegenhaltungen:
- WO-A1-2005/039520
- WO-A1-2006/110271
- DE-A1- 10 234 884

## Beschreibung

Die Erfindung betrifft ein kosmetisches Sonnenschutzprodukt auf Basis von W/Si-Emulsionen mit verbesserter Wirksamkeit.

Der Einbeziehung von organischen Sonnenschutzfiltern in moderne Hautpflegeprodukte auf Silikonölbasis stehen bei einigen Vorteilen auch eine Reihe von Nachteilen gegenüber. In Sonnenschutzzubereitungen mit hohen Sonnenschutzfaktoren (SPF > 15) (SPF = Sun Protection Factor) ist es erforderlich, deutlich mehr organische Sonnenschutzfilter einzubringen, als theoretisch für die Erreichung des entsprechenden SPF erforderlich wäre, da Silikonöle diese Filter nicht leicht aufnehmen und eine den SPF herabsetzende Eigenschaft zeigen. Weiterhin wird es mit höheren Konzentrationen an Filtern schwieriger, eine zufriedenstellende Textur der Emulsion zu erreichen. Das leichte Hautgefühl (touch) geht verloren, Weißeln-Effekte treten auf, und die Weichheit während und nach dem Auftragen des Produktes auf die Haut wird unbefriedigend.

In der DE 102 34 884 A1 ist eine mehrphasige kosmetische Sonnenschutzzubereitung beschrieben, die als Si/W-Emulsion mehr als 50 % Silikonöle in der Fettphase, weiterhin polyethermodifizierte Polysiloxane und UV-Filter enthält, und bei der wenigstens eine der zwei, drei oder vier Phasen nicht durchsichtig oder durchscheinend ist. Diese Mehrphasigkeit wird von der Erfindung nicht angestrebt.

Der Erfindung liegt die Aufgabe zugrunde, ein kosmetisches Sonnenschutzprodukt auf Basis von W/Si-Emulsionen zu entwickeln, das trotz hoher Gehalte an organischen Sonnenschutzfiltern ein sehr gutes Sensorikprofil aufweist und insbesondere ein lang anhaltendes Weichheitsgefühl zeigt.

Erfindungsgemäß wird ein Sonnenschutzprodukt gemäß Anspruch 1 bereitgestellt.

Der Emulgator besteht aus einem Emulgatorgemisch aus anionischen und nicht-ionischen Emulgatoren. Dies sind ausgewählt aus der Gruppe, bestehend aus Fettalkoholsalzen, Fettethersalzen, Fettsäureestern und Gemischen davon. Dabei ist der Fettalkohol ein Behenylalkohol. Die Fettsäureester sind Glyceryl Stearate und Glyceryl Stearate Citrate. Das Fettalkoholsalz ist Natrium-Dicocoylethylendiamin-PEG-15-Sulfate. Mit einem Emulgatorgemisch der genannten Stoffe wird eine vorteilhafte Fixierung der Komponenten des Systems erreicht und damit eine besondere Stabilität der kosmetischen Formel.

Bei der Beurteilung der Sensorikeigenschaften des erfindungsgemäßen Sonnenschutzproduktes sind insbesondere solche zu berücksichtigen, wie Weißeln beim Verteilen auf der Haut, Gleiten des Cremekörpers zwischen den Fingern (Textur), Filmdicke beim Verteilen (Spreiten), Dauer der Verteilung, Klebrigkeit beim Verteilen, Weichheit nach dem Verteilen, Fettigkeitsgefühl nach dem Verteilen, Glanz nach dem Verteilen, pudriges Gefühl nach dem Verteilen und allgemeiner Hautkomfort.

Es wurde gefunden, dass das erfindungsgemäße Sonnenschutzprodukt in der Mehrzahl solcher Sensorikeigenschaften außerordentlich gut ist. Insbesondere sind die Textur, der allgemeine Hautkomfort und das Weichheitsgefühl nach dem Verteilen auf der Haut und lange Zeit danach besser als bei anderen Produkten.

Weiterhin sind besonders hervorzuheben die ausgezeichnete Verteilung der organischen Sonnenschutzfilter auf der Haut innerhalb des erfindungsgemäßen Systems sowie dessen Homogenität auf der Haut.

Die Messung der Viskosität erfolgt mit einem Brookfield-Viskosimeter DV-II+ (Brookfield Engin. Lab., Inc., Stroughton, MA, USA) bei 20 °C, 3000-10000 mPa·s Tellerspindel, >10.000-20.000 Helipath TC/TD-Spindel.

Das erfindungsgemäße Produkt enthält weiterhin die in Anspruch 1 angegebenen kosmetischen Hilfs- und Trägerstoffe.

Erfindungsgemäß enthält das Sonnenschutzprodukt entsprechende wasser- und/oder öllösliche UVA- und UVB-Filter. Dies sind Butyl Methoxydibenzoylmethane, Isoamyl-p-Methoxycinnamate, Ethylhexyltriazone und Bis-Ethylhexyloxyphenol Methoxyphenyl Triazine.

Die Bestimmung des Sonnenschutzfaktors SPF erfolgt üblicherweise nach dem COLIPA-Verfahren (Colipa-Ref.: 94/289 (1994)), wobei im wesentlichen nur UVB-Strahlung erfasst wird. Die *in vivo*-UVA-Schutzfaktoren werden nach dem Persistant Pigment Darkening-Verfahren (PPD) als Endpunkt (Photodermatol. Photoimmunol. Photomed. 16, 245-249 (2000)) ermittelt.

Ein Verfahren, das in der WO 2005/103659 beschrieben ist, berücksichtigt als erstes Verfahren, dass die Ursache jeglicher, durch die gesamte UV-Einwirkung bedingten Hautschädigung die Gesamtzahl der erzeugten freien Radikale ist. Diese Anzahl wird weniger durch die Intensität der UV-Strahlen als vielmehr durch die Gesamt-UV-Dosis beeinflusst. Für diese Gesamtanzahl erzeugter freier Radikale, die mittels ESR genau gemessen werden kann, kann ein integrierter SPF ermittelt werden, der dem Anwender eine exakte Information über den tatsächlichen Schutz gegenüber UV-Strahlung gibt.

Solch ein SPF ist in zweifacher Hinsicht integriert: Er erfasst das gesamte UV-Spektrum, und er erfasst die komplette Hauttiefe, in der Schädigung durch UV-Strahlung auftreten kann.

Ein weiterer Vorteil bei der Messung des integrierten SPF oder ISPF wird dadurch erreicht, dass nicht mehr wie bisher das Auftreten eines Erythems auf der Haut eines Probanden nach einer unter ethischen Gesichtspunkten bedenklichen Bestrahlungsbehandlung gemessen werden muss, sondern die Messung an 2 bis 4 Hautstücken von je 1 cm² aus der Humanchirurgie oder von Hautsubstraten (künstliche Haut) oder direkt an Schweinehaut mit gleicher oder besserer Zuverlässigkeit erfolgen kann. Damit entfallen Strahlenschädigungen der Haut bei den Probanden, weil die Notwendigkeit für Probanden an sich für dieses Messverfahren entfällt. Nach dem COLIPA-Standard werden 10-20 Freiwillige benötigt. Auch die Auswirkungen langer Wartezeiten bis zum Erhalt der Ergebnisse entfallen sowie die Festlegung bestimmter Standards, gegen die bei den bekannten Verfahren auszuwerten ist.

Sowohl gerätetechnisch als auch von der Methodik ist die Bestimmung des ISPF ein erheblich reduzierter Kostenfaktor, da eine RGV-Messung bereits jetzt hinsichtlich der Kosten bei etwa der Hälfte bis einem Drittel der Kosten traditioneller SPF-Bestimmungsverfahren liegt.

Darüber hinaus liegt die Genauigkeit der Messung und Berechnung des ISPF bei etwa ±10 %, während das traditionelle SPF-Messverfahren, d. h. COLIPA (UVB-Schutz), nur bei einer Genauigkeit von ±20-25 % liegt, bei hohen, in verschiedenen Testinstituten gemessenen SPFs sogar nur bei Genauigkeiten von 30-50 %.

Der ISPF der erfindungsgemäßen Sonnenschutzprodukte liegt im Bereich von ISPF 12 - 25. Dabei ist es besonders vorteilhaft, dass der Anteil von UVA-Filtern in der Größenordnung der UVB-Filter liegt, insbesondere bei einem Verhältnis UVA-Filter zu UVB-Filter im Bereich von 1:1,5 bis 1:1.

Die Verwendung des erfindungsgemäßen Sonnenschutzproduktes kann z.B. erfolgen in Sonnencremes, Sonnengelen, After-sun-Produkten, Tagescremes, Nachtcremes, Masken, Sonnenlotionen, Make up's, Lippenstiften, Augenkosmetik, Haarmasken, Haarspülungen, Haarshampoos, Duschgelen, Duschölen, Badeöl. Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist. Umgebungstemperatur bedeutet 18-25 °C.

### Beispiel 1 W/Si Sonnenemulsion SPF 60 (ISPF 20)

| | |
|---|---|
| (1) Cyclopentasiloxane & PEG-12 Dimethicone Crosspolymer (DC 9011) Cyclopentasiloxane & | 16 |
| (2) PEG/PPG-18/18 Dimethicone & Cyclotetrasiloxane (DC5225) | 4 |
| (3) Cyclopentasiloxane & Cyclotetrasiloxane (DC 245) | 10 |
| (4) Deionisiertes Wasser | q.s. ad 100 |
| (5) Divinyldimethicone/Dimethicone Copolymer & C12-13 Pareth-3 & C12-13 Pareth 23 & Cyclotetrasiloxane (HMW 2220) | 1 |
| (6) Methylene Bis-Benzotriazolyl & Tetramethylbutylphenol (Tinosorb M) | 6 |
| (7) Sodiumlaurethsulfate | 1,5 |
| (8) Natriumchlorid | 1,2 |
| (9) Butyl Methoxydibenzoylmethane | 2 |
| (10) Bis-Ethylhexyloxyphenol Methoxyphenyl Triazine | 2 |
| (11) Ethylhexyl Triazone | 2 |
| (12) Isoamyl p-Methoxycinnamate | 10 |
| (13) Konservierungsmittel | 0,6 |
| (14) Palmitoyl Proline & Palmitic Acid & Magnesium Palmitoyl Glutamate & Sodium Palmitoyl Sarcosinate | 0,3 |
| (15) Behenyl Alcohol & Glyceryl Stearate & Glyceryl Stearate Citrate & Sodium Dicocoylethylenediamine PEG-15 Sulfate | 0,3 |
| (16) Alkohol | 4 |
| (17) Parfüm | 0,2 |

Die siliconölhaltigen Komponenten werden bei Umgebungstemperatur vermischt und die öllöslichen Sonnenschutzfilter hinzugegeben. Separat wird Wasser mit den wasserlöslichen Komponenten vermischt. Anschließend werden beide Phasen bei Umgebungstemperatur miteinander vermischt und homogenisiert. Viskosität 5170 mPa·s.

### Beispiel 2 W/Si Sonnenemulsion SPF 50 (ISPF 15)

Es wurde ähnliche Formulierung wie im Beispiel 1 hergestellt mit folgenden Unterschieden
(1) 15 %; (7) 1,5 %; (8) 0,8 %; (11) und (15) jeweils 0 %.
Die Herstellungsweise entsprach der von Beispiel 1.
Viskosität 8530 mPa·s.

### Beispiel 3 W/Si Sonnenemulsion SPF 50 (ISPF 12)

Es wurde ähnliche Formulierung wie im Beispiel 1 hergestellt mit folgenden Unterschieden
(1) 18 %; (2) 3 %; (7) 1 %; (8) 0,8 %; (10) 1 %; (16) 4 %.
Die Herstellungsweise entsprach der von Beispiel 1.
Viskosität 6320 mPa·s.

### Beispiel 3 Sensoriktest

### Teil A

Die folgenden Daten wurden von einer Probandengruppe mit 2x9 Probanden erhoben, die jeweils die Produkte nach Beispiel 1 und einem Vergleichsprodukt nach den folgenden Bewertungskriterien testeten und eine Note zwischen 1 und 9 vergaben. Die genannten Werte sind die Durchschnittswerte von allen 18 Probanden.

| | |
|---|---|
| Produkt nach Beispiel 1 (Erfindung) | : A |
| Vergleichsprodukt | : B |

Das Vergleichsprodukt B bestand aus 50 % flüchtigem Silikonöl, 5 % Cyclodimethicone Copolymer, 2% Aminosäurederivat Amisoft CA, 0,5 % Emulgator Sodium Stearate, 8 % UVA-Filter, 14 % UVB-Filter, Wasser, Konservierungsmittel, Parfüm.
1. Weißeln beim Verteilen auf der Haut
   A=2,4 B=4,8
   Erfindung um 1,8 Punkte besser
2. Gleiten des Cremekörpers zwischen den Fingern (Textur)
   A=8,2 B=3,4
   Erfindung um 4,8 Punkte besser
3. Filmdicke beim Verteilen
   A=4,1 B=2,9
   Erfindung um 1,2 Punkte besser und damit homogener
4. Zeit bis zur endgültigen Verteilung (playtime)
   A=2,2 B=4,2
   Erfindung um 2,0 Punkte besser
5. Klebrigkeit bei Verteilung
   A=2,6 B=2,7
   Beide nahezu gleich
6. Fettigkeitsgefühl nach dem Verteilen
   A=4,0 B=4,8
   Erfindung um 0,8 Punkte besser
7. Glanz nach dem Verteilen
   A=2,1 B=2,0
   Beide nahezu gleich
8. Weichheit der Haut nach dem Verteilen
   A=6,8 B=5,3
   Erfindung um 1,5 Punkte besser
9. Pudriges Gefühl nach dem Verteilen
   A=3,8 B=2,4
   Erfindung um 1,4 Punkte besser
10. Allgemeiner Hautkomfort (Bewertung 3, 5, 7, 8, 9, 10)
   A= +4,1 B=0
   Erfindung um 4,1 Punkte besser

Daraus ergibt sich, dass 8 Eigenschaften besser bewertet wurden und 2 Eigenschaften nahezu gleich. Das erfindungsgemäße Produkt kann daher als deutlich besser als ein vergleichbares Produkt hinsichtlich der Sensorik-Eigenschaften angesehen werden.

### Teil B

Für den Test bezüglich der Weichheit der Haut nach dem Auftragen wurden die Probanden (Gruppe A und B) in folgender zeitlicher Reihenfolge befragt. Die Ziffern nach den Gruppenbuchstaben bedeutet die Anzahl der Probanden für diese Bemerkung)

| **ZEIT** | **BEMERKUNG** | | | | | |
|---|---|---|---|---|---|---|
| | Sehr weich | | weich | | mäßig weich | |
| Sofort nach dem Auftragen | A7 | B5 | A2 | B4 | A0 | - |
| 30 Min. danach | A6 | B5 | A3 | B3 | A0 | B1 |
| 2 Std. danach | A6 | B4 | A2 | B2 | A1 | B2 |
| 4 Std. danach | A4 | B2 | A3 | B3 | A2 | B4 |
| 8 Std. danach | A4 | B0 | A2 | B2 | A3 | B7 |

Während 4 bzw. 2 Probanden der A-Gruppe nach 8 Stunden das Weichheitsgefühl noch als "sehr weich" bzw. "weich" bezeichneten, waren es in der Gruppe B nur 2 Probanden, die "weich" einschätzten. Dies zeigt deutlich die langanhaltende Wirksamkeit des erfindungsgemäßen Produktes.

## Patentansprüche

1. Sonnenschutzprodukt auf Basis einer W/Si-Emulsion, bestehend aus:
| | |
|---|---|
| 16 Gew.-% | Cyclopentasiloxane & PEG-12 Dimethicone Crosspolymer; |
| 4 Gew.-% | Cyclopentasiloxane & PEG/PPG-18/18 Dimethicone & Cyclotetrasiloxane; |
| 10 Gew.-% | Cyclopentasiloxane & Cyclotetrasiloxane; |
| 1 Gew.-% | Divinyldimethicone/Dimethicone Copolymer & C12-13 Pareth-3 & C12-13 Pareth 23 & Cyclotetrasiloxane; |
| 6 Gew.-% | Methylene Bis-Benzotriazolyl & Tetramethyl-butylphenol; |
| 1,5 Gew.-% | Sodiumlaurethsulfate; |
| 1,2 Gew.-% | Natriumchlorid; |
| 2 Gew.-% | Butyl Methoxydibenzoylmethane; |
| 2 Gew.-% | Bis-Ethylhexyloxyphenol Methoxyphenyl Triazine; |
| 2 Gew.-% | Ethylhexyl Triazone; |
| 10 Gew.-% | Isoamyl p-Methoxycinnamate; |
| 0,6 Gew.-% | Konservierungsmittel; |
| 0,3 Gew.-% | Palmitoyl Proline & Palmitic Acid & Magnesium Palmitoyl Glutamate & Sodium Palmitoyl Sarcosinate; |
| 0,3 Gew.-% | Behenyl Alcohol & Glyceryl Stearate & Glyceryl Stearate Citrate & Sodium Dicocoylethylenediamine PEG-15 Sulfate; |
| 4 Gew.-% | Alkohol; |
| 0,2 Gew.-% | Parfüm; |
| und q.s. ad 100 Gew.-% deionisiertes Wasser. | |

## Claims

1. A sun protection product on the basis of W/Si emulsions comprising
| | |
|---|---|
| 16 % by weight | Cyclopentasiloxane & PEG-12 dimethicone crosspolymer; |
| 4 % by weight | Cyclopentasiloxane & PEG/PPG-18/18 dimethicone & Cyclotetrasiloxane; |
| 10 % by weight | Cyclopentasiloxane & cyclotetrasiloxane; |
| 1 % by weight | Divinyldimethicone/dimethicone copolymer & C12-13 Pareth-3 & C12-13 Pareth 23 & cyclotetrasiloxane; |
| 6 % by weigt | Methylene-bisbenzotriazolyl & tetramethylbutylphenol; |
| 1,5 % by weight | Sodium laureth sulfate; |
| 1,2 % by weight | Sodium chloride; |
| 2 % by weight | Butyl-methoxydibenzoylmethane; |
| 2 % by weight | Bis-ethylhexyloxyphenol methoxyphenyltriazine; |
| 2 % by weight | Ethylhexyltriazone; |
| 10 % by weight | Isoamyl p-methoxycinnamate; |
| 0,6 % by weight | Preservative; |
| 0,3 % by weight | Palmitoylproline & palmitic acid & magnesium palmitoylglutamate & sodium palmitoylsarcosinate |
| 0,3 % by weight | Behenyl alcohol & glyceryl stearate & glyceryl stearate citrate & sodium dicocoylethylenediamine PEG-15 sulfate |
| 4 % by weight | Alcohol; |
| 0,2 % by weight | Perfume; |
| and q.s. ad 100 % by weight deionized water. | |

## Revendications

1. Soin solaire protecteur à base d'une émulsion eau/silicone (W/Si), constitué de :
16 % en poids de cylopentasiloxane & polymère PEG 12 diméthicone réticulé,
4 % en poids de cylopentasiloxane & PEG/PPG-18/18 diméthicone & cyclotétrasiloxane,
10 % en poids de cylopentasiloxane & cyclotétrasiloxane,
1 % en poids de copolymère divinyldiméthicone/diméthicone & C12-13 pareth-3 & C12-13 pareth 23 & cyclotétrasiloxane,
6 % en poids de méthylène bis-benzotriazolyl & tétraméthyl-butylphénol,
1,5 % en poids de laurethsulfate de sodium,
1,2 % en poids de chlorure de sodium,
2 % en poids de butyl méthoxydibenzoylméthane,
2 % en poids de bis-éthylhexyloxyphénol méthoxyphényl triazine,
2 % en poids d'éthylhexyl triazone,
10 % en poids d'isoamyl p-méthoxycinnamate,
0,6 % en poids d'agent conservateur,
0,3 % en poids de palmitoyl proline & acide palmitique & palmitoyl glutamate de magnésium & palmitoyl sarcosinate de sodium,
0,3 % en poids d'alcool béhénique & glycéryl stéarate & glycéryl stéarate citrate & sodium dicocoyléthylènediamine PEG-15 sulfate,
4 % en poids d'alcool,
0,2 % en poids de parfum,
et une q.s. d'eau désionisée pour compléter à 100 % en poids.
